# FASCICULE DE BREVET EUROPEEN

(11) **EP 3 544 573 B1**
(45) Date de publication et mention de la délivrance du brevet: **30.09.2026**
(21) Numéro de dépôt: 16815887.1
(22) Date de dépôt: 25.11.2016
(51) Int. Cl.: A61K 8/34, A61Q 3/02, A61K 8/65, A61K 8/81, A61K 8/86, A61K 8/31

(54) **COMPOSITION FILMOGÈNE**
FILMBILDENDE ZUSAMMENSETZUNG
FILM-FORMING COMPOSITION

(43) Date de publication de la demande: 02.10.2019
(73) Titulaire: Urgo Recherche Innovation et Développement, 21300 Chenove (FR)
(72) Inventeur: DE BEER, Andrew, 2729 CK Zoetermeer (NL); OPDEBEEK, Katelijne, 2221 Booischot (BE)
(74) Mandataire: Plasseraud IP
(86) Numéro de dépôt international: PCT/FR2016/053101
(87) Numéro de publication internationale: WO 2018/096224

(56) Documents cités:
- EP-A1- 2 149 367
- EP-A1- 2 583 663
- WO-A1-2009/035970
- WO-A1-91/02538
- WO-A1-91/12793
- US-A1- 2006 165 635
- US-A1- 2007 086 966
- US-A1- 2015 030 643
- SEPPIC: "SEPINOV TM EMT 10", INTERNET CITATION, April 2005 (2005-04-01), XP002463431, Retrieved from the Internet <URL:http://www.in-cosmeticseasterneurope.com/ExhibitorLibrary/19/SEPINOV_EMT_2.pdf> [retrieved on 20080103]

## Description

La présente invention concerne une composition liquide filmogène comprenant au moins un polymère filmogène dérivé de la kératine, au moins un agent hydratant et au moins un tensioactif dans un milieu pharmaceutiquement acceptable. L'invention concerne également un procédé d'obtention d'un film à partir de ladite composition liquide filmogène ainsi que le film susceptible d'être obtenu par ledit procédé. L'invention se rapporte, par ailleurs, à l'utilisation thérapeutique de ladite composition liquide filmogène ou dudit film pour la protection de la peau, des muqueuses ou des phanères. L'invention se rapporte également à une méthode de traitement cosmétique des ongles fragilisés ainsi qu'à l'utilisation de ladite composition liquide filmogène ou dudit film pour améliorer l'aspect et/ou renforcer les ongles fragilisés.

Les compositions filmogènes sont destinées à être appliquées, avec ou sans applicateur, sur des tissus tels que la peau, les phanères (les ongles ou les cheveux), les muqueuses. Elles peuvent également être utilisées comme pansement liquide, et sont alors appliquées sur des plaies, des lésions, des cicatrices, des tissus brûlés et/ou des affections cutanées. Elles sont généralement liquides à l'application et contiennent classiquement un polymère filmogène dissous dans un solvant volatil, typiquement de l'eau ou un alcool. L'évaporation du solvant permet alors la formation d'un film solide protecteur.

Les documents EP 2 149 367 et WO 91/02538 A1 décrivent des compositions pour les ongles. Le document US 2015/030643 concerne des nanoémulsions de silicone aminé qui peuvent être utilisées pour protéger les surfaces contre la salissure ou l'humidification. Le document WO 91/12793 décrit des mascaras. Le document EP 2 583 663 décrit une composition et son utilisation sur les matières kératiniques. Le document US 2007/086966 décrit une composition cosmétique anti-âge. Le document WO 2009/035970 décrit une composition pour lisser les cheveux pour au moins 2-3 mois. Le document US 2006/165635 décrit des formulations de produits cosmétiques contenant de la kératine et leur utilisation sur les matières kératiniques,
L'invention a ainsi pour objet, selon un premier aspect, une composition liquide filmogène comprenant, dans un milieu pharmaceutiquement acceptable,
au moins un polymère filmogène dérivé de la kératine présent dans la composition en une teneur en matières sèches allant de 0,2 % à 1 % en poids, par rapport au poids total de la composition ;
- au moins un agent hydratant choisi dans le groupe constitué par la glycérine en association avec le squalane, présent dans la composition en une teneur en matières sèches allant de 1 % à 10 % en poids; et
- au moins un tensioactif choisi parmi les gommes végétales, les acides polyacryliques, le polyéthylène glycol, les polysorbates, et leurs mélanges, présent dans la composition en une teneur en matières sèches allant de 1 à 10% ;
et la composition étant caractérisée en ce qu'elle ne comprend ni de formaldéhyde, ni de parabène.

Selon un second aspect, l'invention a également pour objet un procédé d'obtention d'un film à partir de la composition liquide filmogène précédemment décrite, comprenant les étapes suivantes :
i. on applique ladite composition sur les ongles fragilisés de manière à former un film uniforme,
ii. on laisse sécher pendant 1 à 5 minutes.

Selon un troisième aspect, l'invention a également pour objet le film susceptible d'être obtenu par le procédé de l'invention.

Selon un quatrième aspect, l'invention a pour objet l'utilisation thérapeutique de la composition liquide filmogène ou du film selon l'invention pour la protection de la peau, des phanères et des muqueuses.

Selon un cinquième aspect, l'invention a pour un objet une méthode de traitement cosmétique des ongles fragilisés comprenant l'application de la composition liquide filmogène selon l'invention sur les ongles, de préférence au moins une fois par jour, plus préférentiellement deux fois par jour.

Selon un dernier aspect, l'invention se rapporte également à l'utilisation de la composition liquide filmogène ou du film selon l'invention pour améliorer l'aspect et/ou renforcer les ongles fragilisés.

En particulier, la composition liquide filmogène selon l'invention est physiologiquement acceptable, c'est à dire qu'elle est non toxique et susceptible d'être appliquée sur les tissus tels que la peau, les plaies, les phanères ou les muqueuses d'êtres humains ou d'animaux et permet la formation d'un film biocompatible.

On entend par composition liquide, au sens de la présente demande, une composition qui s'écoule sous son propre poids.

Les compositions actuellement commercialisées pour le traitement des ongles cassants et fragilisés contiennent des produits agressifs, tels que le formaldéhyde. Aussi, beaucoup d'entre elles nécessitent un dissolvant pour les retirer.

La composition selon la présente invention présente les avantages suivants :
- elle sèche rapidement (moins d'une minute) ;
- elle ne nécessite pas de dissolvant pour être retirée ;
- elle est transparente et mate ;
- elle ne contient ni formaldéhyde, ni parabène ;
- son parfum est agréable.

Une étude clinique sur la composition selon l'invention a permis de mettre en évidence :
- un effet bénéfique sur le dédoublement de l'ongle significatif après 14 et 28 jours d'utilisation ;
- un effet bénéfique sur la finesse de l'ongle significatif après 14 et 28 jours d'utilisation ;
- un effet bénéfique sur la fragilité de l'ongle significatif après 14 et 28 jours d'utilisation ;
- un effet bénéfique durcisseur significatif après 14 et 28 jours d'utilisation ;
- un effet bénéfique sur la couleur de l'ongle après 14 et 28 jours d'utilisation ;
- un effet bénéfique lissant après 14 et 28 jours d'utilisation.

### Polymère filmogène dérivé de la kératine

La composition selon l'invention comprend au moins un polymère filmogène dérivé de la kératine.

Selon la présente invention, on entend par "polymère filmogène", un polymère apte à former à lui seul ou en présence d'un agent auxiliaire de filmification, un film continu et adhérent sur un support, notamment sur la peau ou les phanères.

Dans le cadre de la présente invention, le polymère filmogène est un polymère dérivé de la kératine.

Par « polymère dérivé de la kératine » on entend, au sens de la présente invention, un polymère obtenu par modification de la kératine.

La kératine est une protéine fibreuse insoluble de forme hélicoïdale que l'on trouve principalement dans les phanères des êtres humains et des animaux. Les acides aminés présents dans la kératine varient en fonction de sa provenance. La kératine peut notamment être extraite à partir d'une source animale, telle que notamment des cornes, des sabots, de la laine et des plumes d'animaux ainsi que des membranes de coquilles d'œufs.

Selon un mode de réalisations préféré, le polymère dérivé de la kératine est la kératine hydrolysée.

La kératine hydrolysée peut notamment être obtenue par hydrolyse chimique ou enzymatique de la kératine.

Le polymère dérivé de la kératine peut également être une kératine modifiée, c'est-à-dire de la kératine ou de la kératine hydrolysée qui a été modifiée par une ou plusieurs réactions chimiques telle que notamment une réaction de succinylation, de quaternisation, d'estérification ou d'amidification avec un acide gras, de sulfonylation ou d'oxydation.

Le polymère dérivé de la kératine est présent dans la composition selon l'invention en une teneur en matières sèches allant de 0,2 % à 1 % en poids, par rapport au poids total de la composition.

### Agent hydratant

La composition selon l'invention comprend au moins un agent hydratant choisi dans le groupe constitué par la glycérine en association avec le squalane, présent dans la composition en une teneur en matières sèches allant de 1 % à 10 % en poids.

Par « agent hydratant » on entend, au sens de la présente invention, un agent capable d'augmenter la teneur en eau de la peau ou des phanères.

De préférence, l'agent hydratant est présent dans la composition selon l'invention en une teneur en matières sèches allant de 2 % à 7 % en poids, par rapport au poids total de la composition.

### Tensioactif

La composition selon l'invention comprend au moins un tensioactif.

On entend par tensioactif, un composé qui modifie la tension superficielle entre deux surfaces.

Le tensioactif peut notamment être choisi parmi les gommes végétales, les acides polyacryliques, le polyéthylène glycol, les polysorbates, et leurs mélanges.

Un exemple d'acide polyacrylique utilisable en tant que tensioactif est un copolymère d'un sel de l'acide 2-méthyl-2-[(1-oxo-2-propènyl)amino]-1-propanesulfonique et de l'ester 2-hydroxyéthyle de l'acide propénoïque. Par sel de l'acide 2-méthyl-2-[(1-oxo-2-propènypamino]-1-propanesulfonique, on entend ici tout type de sels connus de l'homme du métier tels que les sels de sodium, de potassium, d'ammonium, de préférence un sel de sodium. Un tel produit est par exemple commercialisé par la société SEPPIC sous la dénomination commerciale SEPINOV EMT 10 .

De préférence, le tensioactif est un copolymère d'un sel de l'acide 2-méthyl-2-[(1-oxo-2-propènyl)amino]-1-propanesulfonique et de l'ester 2-hydroxyéthyle de l'acide propénoïque utilisé seul ou en association avec un polysorbate, de préférence le polysorbate 60.

Le tensioactif est présent en une teneur en matières sèches allant de 1 à 10%, de préférence 2 à 6% en poids par rapport au poids total de la composition.

### Milieu pharmaceutiquement acceptable

La composition selon l'invention comprend un milieu pharmaceutiquement acceptable comprenant au moins un solvant.

Par milieu pharmaceutiquement acceptable, on entend, au sens de la présente demande, un milieu compatible avec la peau.

En particulier, le milieu pharmaceutiquement acceptable comprend un solvant compatible avec le polymère filmogène dérivé de la kératine, de préférence on utilisera un solvant hydro-alcoolique.

Le milieu pharmaceutiquement acceptable peut notamment représenter de 30 à 95% en poids, de préférence de 60 à 90% en poids par rapport au poids total de la composition.

### Polymère filmogène secondaire

La composition selon l'invention peut comprendre, en plus du polymère filmogène dérivé de la kératine, un polymère filmogène secondaire.

Parmi les polymères filmogènes secondaires utilisables dans la composition de la présente invention, on peut citer les polymères synthétiques, de type radicalaire ou de type polycondensat, les polymères d'origine naturelle, et leurs mélanges.

Le polymère filmogène secondaire peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide, le copolymère de vinyl méthyl éther et d'anhydride maléique (PVM/MA), et leurs mélanges.

Comme polymère filmogène secondaire, on peut notamment utiliser la nitrocellulose RS 1/8 sec ; RS [1/4] sec. ; [1/2] sec. ; RS 5 sec. ; RS 15 sec. ; RS 35 sec. ; RS 75 sec. ; RS 150 sec ; AS [1/4] sec. ; AS [1/2] sec. ; SS [1/4] sec. ; SS [1/2] sec. ; SS 5 sec., notamment commercialisées par la société HERCULES, ou la nitrocellulose DHL 120/170 IPA commercialisée par la société NOBEL ; les résines toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Le polymère filmogène secondaire peut notamment être présent dans la composition selon l'invention en une teneur en matières sèches allant de 5 à 25% en poids, de préférence allant de 5 à 10% en poids, par rapport au poids total de la composition.

### Agent auxiliaire de filmification

Pour améliorer les propriétés filmogènes de la composition, un agent auxiliaire de filmification peut avantageusement être ajouté.

L'agent auxiliaire de filmification est, bien évidemment, différent du solvant présent dans le milieu pharmaceutiquement acceptable, du polymère filmogène dérivé de la kératine et du polymère filmogène secondaire.

Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).

Ainsi, la composition peut comprendre, en outre, au moins un agent auxiliaire de filmification choisi parmi un agent plastifiant et/ou un agent de coalescence. En particulier, on peut citer, seuls ou en mélange, les agents plastifiants et agents de coalescence usuels, tels que :
- les alcools gras comme l'octyldodécanol, le 2-butyloctanol, le 2-hexyl décanol, le 2-undécylpentadécanol, l'alcool oléique ;
- les glycols et leurs dérivés, tels que la glycérine, le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les acides gras tels que l'acide oléique, l'acide linoléique, l'acide linolénique ;
- les esters de glycol tels que la triacétine (ou triacétate de glycéryle) ;
- les dérivés de propylène glycol et en particulier le propylène glycol phényléther, le propylène glycol diacétate, le dipropylène glycol éthyléther, le tripropylène glycol méthyléther, le propylène glycol butyléther ;
- les esters d'acides, notamment carboxyliques, tels que les citrates, les phtalates, les adipates, les carbonates, les tartrates, les phosphates, les sébacates et en particulier les esters d'acide monocarboxylique tels que l'isononanoate d'isononyle, l'érucate d'oléyle ou le néopentanoate d'octyl-2-docécyle ;
- les dérivés oxyéthylénés, tels que les huiles oxyéthylénées, notamment les huiles végétales, telles que l'huile de sésame, l'huile de ricin, l'huile d'amande, l'huile de canola, l'huile de noisette, l'huile de pistache, l'huile de lin, l'huile de bourrache, l'huile de chanvre, l'huile de jojoba, l'huile de tournesol, l'huile de germe de blé, l'huile de maïs et/ou de germe de maïs, l'huile d'arachide, l'huile d'avocat, l'huile de carthame, l'huile de colza, l'huile d'olive, l'huile d'argan, l'huile de tournesol, l'huile de pépin de raisin, l'huile de soja, l'huile de noix, l'huile de pépin de de courge, l'huile de palme, l'huile de coprah, et leurs mélanges. L'huile peut également être un dérivé d'une des huiles végétales citées précédemment. Il peut s'agir d'huile hydrogénée ou non, peroxydée ou non ;
et leurs mélanges.

Par exemple, la teneur en agent auxiliaire de filmification peut aller de 0,05 à 5% et en particulier de 0.1 à 3% en poids par rapport au poids total de la composition.

### Additifs

La composition selon l'invention peut comprendre un ou plusieurs additifs pharmaceutiquement acceptables, comme par exemple les parfums, les arômes, les colorants, les pigments, les agents matifiants, les agents rhéologiques, les conservateurs, les vitamines, les huiles essentielles et les agents actifs, notamment choisis parmi les agents anti-bactériens, les antiseptiques, les anti-viraux, les agents antifongiques, les anti-douleurs, les anti-inflammatoires, les agents favorisant la cicatrisation, les agents hydratants, les agents dépigmentants, les agents kératolytiques, les actifs restructurants, les anesthésiques, les filtres solaires, et leurs mélanges.

En particulier, les actifs pouvant être introduits dans la composition selon l'invention peuvent être choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-viraux tels que l'Aciclovir, le Famciclovir, le Ritonavir ;
- les antifongiques tels que les polyènes, le Nystatin, l'Amphotéricine B, la Natamycine, les imidazolés (Miconazole, Ketoconazole, Clotrimazole, Éconazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole, Posaconazole, Voriconazole), les allylamines, la Terbinafine, l'Amorolfine, la Naftifine, la Buténafine ;
- la Flucytosine (antimétabolite), la Griséofulvine, la Caspofungine, la Micafungine ;
- les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation tels que le Rétinol, la Vitamine A, la Vitamine E, la N-acétyl-hydroxyproline, la metformine, les extraits de Centella Asiatica, la papaïne, les silicones, les huiles essentielles de thym, de niaouli, de romarin et de sauge, l'acide hyaluronique, les oligosaccharides polysulfatés synthétiques ayant 1 à 4 unités oses tels que le sel de potassium du sucrose octasulfaté, le sel d'argent du sucrose octasulfaté ou le sucralfate, l'Allantoïne ;
- les agents hydratants tels que l'acide hyaluronique, l'urée, le glycérol, les acides gras, modulateurs des aquaporines, les huiles végétales, le chitosan, certains sucres dont le sorbitol, les beurres et les cires ;
- les agents dépigmentants tels que l'acide kojique (Kojic Acid SL^{®} - Quimasso (Sino Lion)), l'Arbutine (Olevatin^{®} - Quimasso (Sino Lion)), le mélange de palmitoylpropyl de sodium et d'extrait de nénuphar blanc (Sepicalm^{®} - Seppic), l'undécylénoyl phénylalanine (Sepiwhite^{®} - Seppic), l'extrait de réglisse obtenue par fermentation d'Aspergillus et éthoxydiglycol (Gatuline Whitening^{®} - Gattefossé), l'acide octadécènedioïque (ODA White^{®} - Sederma), l'alpha-arbutin (Alpha-arbutin^{®}, SACI-CFPA (Pentapharm)), l'extrait aqueux de feuilles Arctophylos Uva Ursi (Melfade-J^{®} - SACI-CFPA (Pentapharm)), le mélange de plante complexe Gigawhite^{®} (SACI-CFPA (Alpaflor)), la diacétyl boldine (Lumiskin^{®} - Sederma), l'extrait de mandarine du Japon (Melaslow^{®} - Sederma), le mélange d'extrait de citron enrichi en acide citrique et d'extrait de concombre (Uninontan^{®}U-34 - Unipex), le mélange d'extrait de Rumex occidentalis et de vitamine C (Tyrostat^{®} 11 - Unipex), des oligopeptides (Mélanostatine 5^{®} - Unipex), le dipalmitate kojique (KAD-15^{®} - Quimasso (Sino Lion)), le complexe d'origine naturelle Vegewhite^{®} de LCW, des extraits de germe de blé (Clariskin^{®} II - Silab), l'éthyldiamine triacétate (EDTA);
- les agents kératolytiques tels que l'acide salicylique, le salicylate de zinc, l'acide ascorbique, les acides alpha hydroxylés (acide glycolique, lactique, malique, citrique, tartrique), les extraits d'Erable argenté, de Griottier, de Tamarinier, l'urée, le rétinoïde topique Kératoline^{®} (Sederma), les protéases obtenues par fermentation de Bacillus Subtilis, le produit Linked-Papain^{®} (SACI-CFPA), la papaïne (enzyme protéolytique issue du fruit de papaye);
- les actifs restructurants (par exemple resctructurants des phanères) tels que les dérivés de silice, la vitamine E, la camomille, le calcium, l'extrait de prêle, le Lipester de soie;
- les anesthésiques tels que la benzocaïne, la lidocaïne, la dibucaïne, le chlorhydrate de pramoxine, la bupivacaïne, la mepivacaïne, la prilocaïne, l'étidocaïne ;
- les filtres solaires, tels que les filtres chimiques (Oxybenzone, Sulisobenzone, Dioxybenzone, Tinosorb S^{®}, Avobenzone, p-méthoxycinnamate de 2-éthoxyéthyle, Uvinul^{®} A+, Mexoryl^{®} XL, Méthoxycinnamate ou octinoxate d'octyle, Salicylate ou octisalate d'octyle, octyl triazone ou Uvinul^{®} T 150, salicylate de méthyle, meradimate, enzacamène, MBBT ou Tinosorb^{®} M, cyanophénylcinnamate d'octyle ou Parsol^{®} 340, Acide para-aminobenzoïque, Ensulizole, Parsol^{®} SLX ou Polysiloxane-15 ou Benzylidène malonate polysiloxane, salicylate de triéthanolamine ou salicylate de trolamine, Mexoryl^{®} SX ou acide téréphtalylidène dicamphosulfonique) et les filtres minéraux (oxydes de Zinc, dioxyde de titane, kaolin, ichtyol).

### Préparation d'un film

Selon un mode particulier de réalisation, l'invention a pour objet un procédé d'obtention d'un film à partir d'une composition filmogène telle que décrite précédemment, comprenant les étapes suivantes :
i. on applique une composition telle que décrite précédemment sur les ongles fragilisés de manière à former un film liquide uniforme,
ii. on laisse sécher pendant 1 à 5 minutes.

L'épaisseur du film liquide uniforme est par exemple supérieure à 500 nm, de préférence comprise entre 1 et 300 µm, plus préférentiellement comprise entre 2 et 200 µm.

L'invention a aussi pour objet un film susceptible d'être obtenu à partir du procédé décrit ci-dessus.

### Utilisation thérapeutique pour la protection de la peau, des muqueuses ou des phanères

Selon un mode particulier de réalisation, l'invention a pour objet la composition filmogène ou le film tels que décrits précédemment, pour leur utilisation pour la protection de la peau, des muqueuses ou des phanères.

Ainsi, la composition ou le film selon l'invention peuvent notamment être utilisés en tant que pansement liquide, de préférence pour la protection des plaies, des lésions, des cicatrices, des tissus brûlés et/ou des affections cutanées.

### Méthode de traitement cosmétique des ongles

L'invention a également pour objet une méthode de traitement cosmétique des ongles fragilisés comprenant l'application de la composition précédemment décrite sur les ongles, de préférence au moins une fois par jour, plus préférentiellement deux fois par jour. Préférentiellement, l'application sur les ongles se fait sur une durée d'au moins sept jours, de préférence au moins quatorze jours.

La méthode cosmétique de l'invention permet notamment d'améliorer l'aspect et/ou renforcer les ongles fragilisés
Ainsi, l'invention a également pour objet l'utilisation de la composition filmogène ou du film tels que décrits précédemment pour améliorer l'aspect et/ou renforcer les ongles fragilisés.

L'invention va être décrite plus en détails à l'aide des exemples suivants qui sont donnés à titre purement illustratif et non limitatif.

### Exemple 1

On a préparé la composition suivante :

| | | **Example 1** |
|---|---|---|
| **Nom commercial** | **Nom INCI** | **% massique** |
| | eau | 33.71 |
| | alcool dénaturé | 54.72 |
| Sepinov EMT 10 commercialisé par la société SEPPIC | copolymère d'un sel de sodium de l'acide 2-méthyl-2-[(1-oxo-2-propènyl)amino]-1-propanesulfonique et de l'ester 2-hydroxyéthyle de l'acide propénoïque | 3.75 |
| | glycérine | 3 |
| | squalane | 2.55 |
| | Polysorbate 60 | 0.55 |
| | Kératine hydrolysée | 0.525 |
| | phénoxyethanol | 0.09 |
| | sorbate de potassium | 0.09 |
| | benzoate de sodium | 0.015 |
| | parfum | 1 |

La composition selon l'exemple 1 a été préparée en mélangeant l'eau et l'alcool, puis on ajoute la kératine hydrolysée, la glycérine, le squalane, le polysorbate 60, le phénoxyéthanol, le sorbate de potassium, le benzoate de sodium puis le parfum jusqu'à obtention d'un mélange homogène.

### Exemple 2 : Evaluation de l'efficacité de la composition selon l'exemple 1 pour traiter les ongles fragilisés

22 femmes volontaires âgées entre 22 et 68 ans présentant des ongles fragilisés (c'est-à-dire cassants, dédoublés, fins ou mous sans lien avec une pathologie) ont testé le produit selon l'exemple 1.

Ce produit était appliqué sur les ongles deux fois par jour pendant 28 jours.

L'objectif de l'évaluation est :
- d'évaluer l'efficacité du produit étudié sur l'apparence des ongles fragilisés ;
- d'évaluer son effet réparateur et fortifiant / durcissant ;
- d'évaluer subjectivement, ses caractéristiques organoleptiques et son efficacité ;
- d'évaluer la rémanence du produit, par les volontaires, 14 jours après l'arrêt des soins.

Les critères d'évaluations sont :
- scorage clinique par le dermatologue (à l'aide d'échelles structurées) ;
- mesure des variations de la Perte Insensible en Eau (Aquaflux^{®}) ;
- recueil des effets indésirables.

Ces critères sont évalués avant le traitement (J0), 14 jours après le début du traitement (J14) et 28 jours après le début du traitement (J28).

Les critères d'évaluations suivants sont également déterminés :
- évaluation subjective (questionnaire) à 72 h après la 1ère application (t72h), 7 jours après le début du traitement (J7), 14 jours après le début du traitement (J14), 28 jours après le début du traitement (J28) et 42 jours après le début du traitement (J42) ;
- mesure de l'épaisseur de l'ongle (DermaScan C^{®}) à J0 et J28.

### 1. Scorage visuel

Le dermatologue responsable de l'évaluation a réalisé un scorage visuel de l'état des ongles (scorage de l'ongle le plus atteint sur la main traitée) en début d'étude avant application du produit puis après 14 jours de soins et en fin d'étude après 28 jours de soins. Il a évalué le dédoublement de l'ongle, la finesse de l'ongle, la fragilité de l'ongle, si l'ongle était mou, la couleur de l'ongle, si l'ongle était strié.

### 2. Mesure de l'effet réparateur

Le dermatologue a également évalué l'effet réparateur de la composition selon l'exemple 1 en mesurant les variations de la Perte Insensible en Eau (PIE). Lorsque la barrière cutanée et la plaque unguéale sont lésées, il apparaît des dérèglements dans la régulation des échanges d'eau. L'eau migre alors plus facilement vers le milieu extérieur ce qui augmente la Perte Insensible en Eau. Par contre, si l'état de la barrière cutanée et de la plaque unguéale s'améliore, les valeurs de la perte en eau diminuent car la régulation des échanges d'eau est assurée de façon correcte.

La PIE est calculée par évaporation en déterminant le gradient de pression de la couche de vapeur d'eau. Cette technique permet d'évaluer plus particulièrement les effets réparateurs d'un produit.

Les mesures de PIE sont effectuées à l'aide d'une chambre à condensateur Aquaflux^{®} AF100 (Biox Systems Ltd.).

La chambre de mesure de l'Aquaflux^{®} est un cylindre creux. Son extrémité inférieure agit en tant qu'orifice de mesure qui est placé en contact avec la surface d'essai (orifice de 2.6mm). Un adaptateur spécifique pour l'utilisation de l'appareil sur les ongles est utilisé. Son extrémité supérieure est scellée avec un condensateur d'aluminium. Celle-ci est maintenue à une température de -7,6°C. Lorsqu'une mesure est réalisée, la chambre est scellée et l'air à l'intérieur est protégé des flux d'air ambiant. L'Aquaflux^{®} mesure la vitesse d'évaporation d'une substance volatile (dans le cas présent, de l'eau). Cette valeur est exprimée en g/m²/h.

Les mesures sont dupliquées (une mesure sur deux ongles différents) sur la main traitée. Les ongles mesurés sont choisis en fonction de la morphologie des ongles de chaque volontaire, afin de faire les mesures sur les ongles les plus gros et les plus plats possible.

Cette mesure est réalisée avant la première application du produit puis après 14 et 28 jours de soins.

### 3. Epaisseur de l'ongle

Les mesures sont réalisées directement in vivo, à l'aide d'un échographe haute fréquence : le DermaScan C^{®} 2D.

Le principe de la mesure est celui de l'échographie : un faisceau ultrasonore est émis par une céramique piézo-électrique. Ce faisceau est partiellement réfléchi par les interfaces séparant deux milieux d'impédance ultrasonore différente.

L'échographe utilisé est muni d'une sonde de 20 MHz. La sonde est appliquée directement sur la peau. Un gel de contact permet une diffusion homogène du signal.

Cette méthode permet une visualisation bidimensionnelle du plateau au lit de l'ongle et la mesure de son épaisseur (en mm).

Cette mesure est réalisée avant la première application du produit puis après 28 jours de soins.

### 4. Questionnaire d'évaluation subjective

Un questionnaire est rempli par les volontaires à t72h, J7, J14, J28 et J42 afin d'évaluer subjectivement les caractéristiques du produit étudié, son efficacité globale ainsi que son utilisation ultérieure.

### 5. Résultats

Le produit selon l'exemple 1 présente à l'examen clinique dermatologique :
- un effet bénéfique sur le dédoublement de l'ongle significatif après 14 et 28 jours d'utilisation ;
- un effet bénéfique sur la finesse de l'ongle significatif après 14 et 28 jours d'utilisation ;
- un effet bénéfique sur la fragilité de l'ongle significatif après 14 et 28 jours d'utilisation ;
- un effet bénéfique durcisseur significatif après 14 et 28 jours d'utilisation ;
- un effet bénéfique sur la couleur de l'ongle après 14 et 28 jours d'utilisation ;
- un effet bénéfique lissant après 14 et 28 jours d'utilisation.

Les mesures objectives mettent en évidence pour le produit selon l'exemple 1 :
- un effet réparateur objectivé par une diminution significative de la PIE après 4 semaines de soins ;
- une augmentation significative de l'épaisseur des ongles mesurée par le DermaScan^{®} après 4 semaines de soins traduisant des ongles plus solides, plus forts.

Le produit selon l'exemple 1 est apprécié par la majorité des volontaires :
- Dès 3 jours d'application, 59% des volontaires rapportent une amélioration de l'aspect de leurs ongles traités par le produit à l'essai, plus de la moitié des volontaires (55%) trouvent leurs ongles mieux nourris et protégés, 46% trouvent leurs ongles fortifiés, 45% les trouvent mieux hydratés et moins cassants, 32% les trouvent plus durs et 27% moins dédoublés et réparés. Le produit est donc efficace rapidement.
- Dès le 7ème jour d'application, 77% des volontaires notent une amélioration de l'aspect de leurs ongles l'effet du produit testé étant optimal sur la majorité des paramètres étudiés avec 73% des volontaires trouvant leurs ongles mieux nourris et fortifiés, 68% mieux hydratés et protégés et 64% moins cassants. Après une semaine de traitement l'aspect des ongles est amélioré chez plus de 3 volontaires sur 4. Dès 7 jours d'application, les ongles sont mieux nourris, fortifiés, hydratés, protégés et moins cassants chez plus de 60% des volontaires.
- Après 2 semaines d'application, 82% des volontaires notent une amélioration de l'aspect de leurs ongles, l'effet du produit testé étant optimal sur le paramètre dureté de l'ongle, 69% des volontaires trouvant leurs ongles plus durs. Après deux semaines de traitement l'aspect des ongles est amélioré chez plus de 8 volontaires sur 10. Les ongles sont plus durs chez 7 volontaires sur 10.
- Après 28 jours de soins, les ongles traités par le produit sont mieux nourris (69% des sujets), plus hydratés (68% des sujets), réparés (69% des sujets), fortifiés (72% des sujets), plus durs (69% des sujets), moins cassants (60% des sujets) et moins dédoublés (59% des sujets). Selon 87% des sujets, l'aspect de leurs ongles s'est amélioré à J28 par rapport à J0 en comparaison à la zone non traitée.
- L'efficacité du produit sur l'amélioration de l'état des ongles est toujours visible 14 jours après l'arrêt des soins ; En effet, 73% des volontaires constatent encore une amélioration de l'aspect de leurs ongles comparé à leur aspect lors du dernier jour de soin attestant d'une rémanence de l'effet bénéfique et 82% des sujets trouvent que l'aspect de leurs ongles est encore amélioré en comparaison à leurs ongles non traités à J42.

## Revendications

1. Composition liquide filmogène **caractérisée en ce qu'**elle comprend, dans un milieu pharmaceutiquement acceptable,
- au moins un polymère filmogène dérivé de la kératine présent dans la composition en une teneur en matières sèches allant de 0,2 % à 1 % en poids, par rapport au poids total de la composition ;
- au moins un agent hydratant choisi dans le groupe constitué par la glycérine en association avec le squalane, présent dans la composition en une teneur en matières sèches allant de 1 % à 10 % en poids; et
- au moins un tensioactif choisi parmi les gommes végétales, les acides polyacryliques, le polyéthylène glycol, les polysorbates, et leurs mélanges, présent dans la composition en une teneur en matières sèches allant de 1 à 10% ;
et la composition étant **caractérisée en ce qu'**elle ne comprend ni de formaldéhyde, ni de parabène.

2. Composition selon la revendication 1, **caractérisée en ce que** le polymère filmogène dérivé de la kératine est la kératine hydrolysée.

3. Composition selon la revendication 1 ou 2, **caractérisée en ce que** l'agent hydratant est présent en une teneur en matières sèches allant de 2 % à 7 % en poids, par rapport au poids total de la composition.

4. Composition selon l'une quelconque des revendications 1 à 3, **caractérisée en ce que** le tensioactif est présent en une teneur en matières sèches allant de 2 à 6% en poids, par rapport au poids total de la composition.

5. Composition selon l'une quelconque des revendications 1 à 4, **caractérisée en ce que** le tensioactif est un copolymère d'un sel de l'acide 2-méthyl-2-[(1-oxo-2-propènyl)amino]-1-propanesulfonique et de l'ester 2-hydroxyéthyle de l'acide propénoïque utilisé seul ou en association avec un polysorbate, de préférence le polysorbate 60.

6. Procédé d'obtention d'un film, caractérisé en que :
i. on applique une composition selon l'une quelconque des revendications 1 à 5 sur les ongles fragilisées de manière à former un film liquide uniforme,
ii. on laisse sécher pendant 1 à 5 minutes.

7. Film susceptible d'être obtenu à partir du procédé selon la revendication 6.

8. Film selon la revendication 7 pour son utilisation pour la protection de la peau, des phanères et des muqueuses.

9. Méthode de traitement cosmétique des ongles fragilisés comprenant l'application de la composition selon l'une quelconque des revendications 1 à 5 sur les ongles, de préférence au moins une fois par jour, plus préférentiellement deux fois par jour.

10. Méthode de traitement cosmétique selon la revendication 9 dans laquelle la composition est appliquée sur les ongles pendant une durée d'au moins sept jours, de préférence au moins quatorze jours.

11. Utilisation de la composition selon l'une quelconque des revendications 1 à 5 ou du film selon la revendication 7 pour améliorer l'aspect et/ou renforcer les ongles fragilisés.

## Patentansprüche

1. Flüssige filmbildende Zusammensetzung, **dadurch gekennzeichnet, dass** sie in einem pharmazeutisch annehmbaren Medium Folgendes umfasst:
- mindestens ein von Keratin abgeleitetes filmbildendes Polymer, das in der Zusammensetzung in einem Trockenmaterialgehalt im Bereich von 0,2 bis 1 Gew.-%, bezogen auf das Gesamtgewicht der Zusammensetzung, vorliegt;
- mindestens ein Feuchthaltemittel ausgewählt aus der Gruppe bestehend aus Glycerin in Kombination mit Squalan, das in der Zusammensetzung in einem Trockenmaterialgehalt von 1 Gew.-% bis 10 Gew.-% vorliegt; und
- mindestens ein Tensid, ausgewählt aus pflanzlichen Gummis, Polyacrylsäuren, Polyethylenglykol, Polysorbaten und Mischungen davon, das in der Zusammensetzung in einem Trockenmaterialgehalt im Bereich von 1 bis 10 % vorliegt; und wobei die Zusammensetzung **dadurch gekennzeichnet ist, dass** sie weder Formaldehyd noch Paraben enthält.

2. Zusammensetzung nach Anspruch 1, **dadurch gekennzeichnet, dass** das von Keratin abgeleitete filmbildende Polymer hydrolysiertes Keratin ist.

3. Zusammensetzung nach Anspruch 1 oder 2, **dadurch gekennzeichnet, dass** das Feuchthaltemittel in einem Trockenmaterialgehalt im Bereich von 2 bis 7 Gew.-%, bezogen auf das Gesamtgewicht der Zusammensetzung, vorliegt.

4. Zusammensetzung nach einem der Ansprüche 1 bis 3, **dadurch gekennzeichnet, dass** das Tensid in einem Trockenmaterialgehalt im Bereich von 2 bis 6 Gew.-%, bezogen auf das Gesamtgewicht der Zusammensetzung, vorliegt.

5. Zusammensetzung nach einem der Ansprüche 1 bis 4, **dadurch gekennzeichnet, dass** das Tensid ein Copolymer eines Salzes der 2-Methyl-2-[(1-oxo-2-propenyl)amino]-1-propansulfonsäure und des 2-Hydroxyethylesters der Propensäure ist, das allein oder in Kombination mit einem Polysorbat, vorzugsweise Polysorbat 60, verwendet wird

6. Verfahren zum Erhalten eines Films, **dadurch gekennzeichnet, dass**:
i. eine Zusammensetzung nach einem der Ansprüche 1 bis 5 auf die angegriffenen Nägel in einer Weise aufgebracht wird, um einen gleichmäßigen flüssigen Film zu bilden,
ii. 1 bis 5 Minuten trocknen gelassen wird.

7. Film, der nach dem Verfahren gemäß Anspruch 6 erhältlich ist.

8. Film nach Anspruch 7 zur Verwendung zum Schutz der Haut, der Hautanhangsgebilde und der Schleimhäute.

9. Kosmetisches Behandlungsverfahren für angegriffene Nägel, umfassend Aufbringen der Zusammensetzung nach einem der Ansprüche 1 bis 5 auf die Nägel, vorzugsweise mindestens einmal täglich, besonders bevorzugt zweimal täglich.

10. Kosmetisches Behandlungsverfahren nach Anspruch 9, bei dem die Zusammensetzung für einen Zeitraum von mindestens sieben Tagen, vorzugsweise mindestens vierzehn Tagen auf die Nägel aufgebracht wird.

11. Verwendung der Zusammensetzung nach einem der Ansprüche 1 bis 5 oder des Films nach Anspruch 7 zur Verbesserung des Erscheinungsbildes und/oder zum Stärken angegriffener Nägel.

## Claims

1. Film-forming liquid composition, **characterized in that** it comprises, in a pharmaceutically acceptable medium,
- at least one keratin-derived film-forming polymer present in the composition in a solids content ranging from 0.2% to 1% by weight, relative to the total weight of the composition;
- at least one moisturizing agent chosen from the group consisting of glycerol in combination with squalane, present in the composition in a solids content ranging from 1% to 10% by weight; and
- at least one surfactant chosen from plant gums, polyacrylic acids, polyethylene glycol, polysorbates, and mixtures thereof, present in the composition in a solids content ranging from 1% to 10%;
and the composition being **characterized in that** it comprises neither formaldehyde nor paraben.

2. Composition according to Claim 1, **characterized in that** the keratin-derived film-forming polymer is hydrolyzed keratin.

3. Composition according to Claim 1 or 2, **characterized in that** the moisturizing agent is present in a solids content ranging from 2% to 7% by weight, relative to the total weight of the composition.

4. Composition according to any one of Claims 1 to 3, **characterized in that** the surfactant is present in a solids content ranging from 2% to 6% by weight, relative to the total weight of the composition.

5. Composition according to any one of Claims 1 to 4, **characterized in that** the surfactant is a copolymer of a 2-methyl-2-[(1-oxo-2-propenyl)amino]-1-propanesulfonic acid salt and of the 2-hydroxyethyl ester of propenoic acid used alone or in combination with a polysorbate, preferably polysorbate 60.

6. Process for obtaining a film, **characterized in that**:
i. a composition according to any one of Claims 1 to 5 is applied to embrittled nails in such a way as to form a uniform liquid film,
ii. it is left to dry for 1 to 5 minutes.

7. Film obtainable from the process according to Claim 6.

8. Film according to Claim 7, for use thereof in protecting the skin, the skin appendages and the mucous membranes.

9. Method for the cosmetic treatment of embrittled nails, comprising the application of the composition according to any one of Claims 1 to 5 to the nails, preferably at least once a day, more preferentially twice a day.

10. Cosmetic treatment method according to Claim 9, in which the composition is applied to the nails for a period of at least seven days, preferably at least fourteen days.

11. Use of the composition according to any one of Claims 1 to 5 or of the film according to Claim 7, for improving the appearance of and/or reinforcing embrittled nails.
